# EUROPEAN PATENT SPECIFICATION

(11) **EP 2 370 805 B1**
(45) Date of publication and mention of the grant of the patent: **27.02.2019**
(21) Application number: 09835846.8
(22) Date of filing: 23.12.2009
(51) Int. Cl.: G01N 21/65, G01N 33/543

(54) **PROGRAMMED SURFACE ENHANCED SPECTROSCOPY PARTICLES**
PROGRAMMIERTE PARTIKEL FÜR OBERFLÄCHENVERSTÄRKTE SPEKTROSKOPIE
PARTICULES PROGRAMMÉES DE SPECTROSCOPIE EXALTÉE DE SURFACE

(30) Priority: 24.12.2008 US 140806 P; 27.05.2009 US 181598 P
(43) Date of publication of application: 05.10.2011
(73) Proprietor: SICPA HOLDING SA, 1008 Prilly (CH)
(72) Inventor: NATAN, Michael, J., Los Altos, California 94024 (US)
(74) Representative: Carvajal y Urquijo, Isabel
(86) International application number: PCT/US2009/069453
(87) International publication number: WO 2010/075534

(56) References cited:
- US-A1- 2002 001 571
- US-A1- 2002 142 480
- US-A1- 2003 166 297
- US-A1- 2006 054 506

## Description

### TECHNICAL FIELD

The present invention relates to programmable SES particles and methods for marking a material. The SES particles of the invention are defined in independent claims 1 and 3. The method of the present invention is defined in independent claim 4.

### BACKGROUND

Certain spectroscopy techniques feature the enhancement of a spectroscopic signal through electromagnetic interaction at a surface. Representative surface enhanced spectroscopic techniques include, but are not limited to surface enhanced Raman spectroscopy (SERS) and surface enhanced resonance Raman spectroscopy (SERRS). In SERS or SERRS, a metal or other enhancing surface will couple electromagnetically to incident electromagnetic radiation and create a locally amplified electromagnetic field that leads to 10⁵- to 10⁹-fold or greater increases in the Raman scattering of a SERS active molecule situated on or near the enhancing surface. The output in a SERS experiment is the fingerprint-like Raman spectrum of the SERS active molecule.

SERS and other SES techniques can be implemented with particles such as nanoparticles. For example, gold is a SERS enhancing surface, and gold colloid may be suspended in a mixture to provide for enhanced Raman spectrum detection. SERS may also be performed with more complex SERS-active nanoparticles, for example SERS nanotags, as described in US Patents No. 6,514,767, No. 6,861,263, No. 7,443,489 and elsewhere. In a SERS nanotag, a reporter molecule is adsorbed to a SERS-active surface, and both the SERS-active surface and the reporter are encapsulated, typically with silica or another relatively impervious material. One advantage of a silica coating is that it prevents the adsorbed molecule from diffusing away. The coating or shell also prevents other molecules from adsorbing to the enhancing surface or particle core. This configuration imparts a level of robustness and environmental insensitivity to the particles that is, for many applications, a desirable feature.

Environmental insensitivity and robustness also cause a SERS nanotag to be spectroscopically static. In particular, a SERS nanotag will return the same signal virtually no matter how long the tag has been applied to an item or imbedded in a substance and whether many types of compound or solution are contacted with the SERS nanotag. Thus the spectroscopic signature of a SERS nanotag typically cannot be changed as a predictable function of time nor can it be altered at the discretion of a tag user. The embodiments disclosed herein are directed toward overcoming this or other problems associated with known surface enhanced spectroscopy particles.

### SUMMARY

The particle of the present invention comprises a programmable surface-enhanced spectroscopy (SES) particle having a SES-active surface and a programmable reporter associated with the SES surface. A programmable SES particle is referred to herein as a PSP. The SES-active surface may, in selected embodiments, be a nanoparticle which may have a diameter between 2 nm and 2000 nm.

In embodiments where the SES-active surface is a nanoparticle, the surface may be of Au, Ag, Cu, Al, Pd, Pt, or a mixture of Au, Ag, Cu, Pd, Pt or Al or any other spectroscopically enhancing surface. The SES metal does not have to be a metal, as non-metallic materials have been shown to be SES-active at certain excitation wavelengths. The SES-active surface may be spherical, or nearly spherical, or have spherical symmetry, or have any other shape.

The programmable SES particles of the invention include a programmable reporter. The programmable reporter provides that the SES particle will return a controlled but variable signal in response to spectroscopic interrogation. The spectroscopic signal can be triggered to change externally or the signal may naturally vary over time. For example, the spectroscopic signal obtained from a programmable SES particle may increase or decrease. Signal change may occur linearly over time. Alternatively, the change in signal over time can assume an infinite variety of forms. The signal may change in response to a trigger and then return to its initial value over time, alternatively the signal obtained from a programmable SES particle may change in response to a trigger and achieve a new constant value. In alternative embodiments of programmable SES particles, the signal obtained from a particle may change without activation by a trigger.

The programmable reporter of a programmable SES particle include a SES-active reporter molecule associated with the SES-active surface. The SES-active reporter may be any substance that returns a spectroscopic signature upon optical interrogation at an appropriate wavelength and in particular a substance that returns an enhanced spectroscopic signal when the SES-active reporter is associated with an SES-active surface.

The various embodiments of SES-active particles may be activated or deactivated by allowing an outside environmental factor or reagent to interfere with the SES-active reporter or SES-active surface. Alternatively, the activity of the SES-active reporter or SES-active surface may be made to be variable over time. For example, selected SES-active reporter substances may be associated with an SES-active surface which reporters decay or change to a non-SES-active material over time. Alternatively, a reagent may be provided which acts upon the SES-active reporter or SES-active surface increasing or decreasing the spectroscopic activity of the particle.

In selected embodiments access to the SES-active reporter and SES-active surface is provided by a porous outer shell surrounding the reporter and surface. Representative examples of a porous outer shell include but are not limited to aerogels, a permselective polymer or a porous silica coating.

Alternatively, an outer shell may be provided which has variable permeability surrounding the SES-active reporter and the SES-active surface. Representative examples of materials having variable permeability include, but are not limited to, thermally depolymerizable polymers, thermally depolymerizable tertiary polycarbonates, thermally responsive polymers, organic-soluble reversed micelles and biodegradable materials. The outer shell may have variable permeability that varies in response to light, heating, cooling, mechanical action or a chemical reagent. Alternatively, the outer shell having variable permeability may be made of a substance which will dissolve or dissociate in use.

The programmable SES particle of the invention as also include a delivery layer associated with the reporter and/or SES-active surface. The particle of the invention is encapsulated with an outer shell. The delivery layer includes a material that will act upon and change the SES activity of the SES-active reporter or the SES-active surface. In selected embodiments the delivery layer may include a material that produces a reagent in response to a stimulus such as heat, cold, light, mechanical stimulus or chemical stimulus. For example, the delivery layer may include, but is not limited to, a thermally depolymerizable polymer, a photoacid generator, a photosensitive compound or a photosensitive polymer.

Selected embodiments of programmable SES particles may also include a diffusion layer typically, but not exclusively, positioned between a delivery layer and the SES-active reporter or SES-active surface. A diffusion layer serves to slow the interaction of a reagent produced by the delivery layer or an outside agent with the reporter or SES-active surface.

### BRIEF DESCRIPTION OF THE DRAWINGS

Fig. 1a - Fig. 1c are graphic representations of the SES signals obtained from selected types of PSPs as a function of time;
Fig. 2a - Fig. 2c are schematic representations of representative PSP configurations;
Fig. 3 is a schematic representation of a PSP including a reverse micelle outer shell;
Fig. 4 is a graphic representation of the strength of the spectroscopic signal obtained from selected PSPs over time;
Fig. 5 is a graphic representation of the strength of the spectroscopic signal obtained from selected PSPs in the presence of peroxide, over time;
Fig. 6 is a graphic representation of the strength of the spectroscopic signal obtained from selected PSPs in the presence of various triggers over time;
Fig. 7 is a graphic representation of the strength of the spectroscopic signal obtained from selected PSPs over time showing the rapid chemical deactivation of a PSP;
Fig. 8 is a graphic representation of the strength of the spectroscopic signal obtained from selected PSPs showing thermal activation;
Fig. 9 is a graphic representation of the strength of the spectroscopic signal obtained from selected PSPs showing thermal deactivation;
Fig. 10 is a graphic representation of the strength of the spectroscopic signal obtained from selected PSPs showing photo-deactivation;
Fig. 11 is a graphic representation of the strength of the spectroscopic signal obtained from selected PSPs over time in the presence of multiple stiggers;
Fig. 12 is a graphic representation of the strength of the spectroscopic signal obtained from a porous PSPs showing rapid activation in the presence of a SES active substance
Fig. 13 is a TEM image of a representative PSP as disclosed herein.

### DESCRIPTION

Unless otherwise indicated, all numbers expressing quantities of ingredients, dimensions reaction conditions and so forth used in the specification and claims are to be understood as being modified in all instances by the term "about".

In this application and the claims, the use of the singular includes the plural unless specifically stated otherwise. In addition, use of "or" means "and/or" unless stated otherwise. Moreover, the use of the term "including", as well as other forms, such as "includes" and "included", is not limiting. Also, terms such as "element" or "component" encompass both elements and components comprising one unit and elements and components that comprise more than one unit unless specifically stated otherwise.

The embodiments disclosed herein relate to programmable particles that are spectroscopically active. In particular, the disclosed particles and methods are surface-enhanced spectroscopy (SES) active. Representative SES techniques include but are not limited to SERS, SERRS and others. Surface enhancement in various other spectroscopy methods or systems has been observed. The most widely studied have been surface-enhanced Raman scattering and surface-enhanced fluorescence (SEF). But a variety of other surface enhanced phenomena have been observed including surface-enhanced hyper Raman scattering (SEHRS), surface-enhanced hyper Raman resonance scattering (SEHRRS), surface-enhanced Rayleigh scattering, surface-enhanced second harmonic generation (SHG), surface-enhanced infrared absorption reflectance (SEIRA), and surface-enhanced laser desorption ionization (SELDI). These are part of a wider field known as plasmon enhancement or plasmon-enhanced spectroscopy, which in addition to the phenomena mentioned above includes surface plasmon enhanced emission (such as SPASERS - surface plasmon amplification of spontaneous emission of radiation), plasmon enhanced diffraction, and plasmon enhanced optical transmission. Plasmon enhancement is also a method to increase the efficiency of solar cells. As used throughout this disclosure SES includes the above listed and any related or similar spectroscopic technique.

Many of the examples herein are described with respect to SERS. It must be noted however that the methods, compositions and particles disclosed herein are equally applicable to SERRS, SEHRS, SEF, SEHRRS, SHG, SEIRA, SPASERS, or other surface enhanced or plasmon enhanced SES technique.

As noted above, one type of known SERS-active nanoparticle is a SERS nanotag, as described in US Patents No. 6,514,767, No. 6,861,263, No. 7,443,489 and elsewhere. In the conventional SERS nanotag composition, a reporter molecule is adsorbed to a SERS-active surface, and both the SERS-active surface and the reporter are encapsulated, typically with silica. One advantage of a silica coating is that it prevents the adsorbed molecule from diffusing away, and also prevents other molecules from adsorbing to the surface. This imparts a level of robustness and environmental insensitivity to the SERS nanotag particles that is, for many applications, a desirable feature.

In certain circumstances however, it is desirable to have the characteristic SERS signature of a particle selectively change. For example, it may be useful to fabricate an embodiment of a SERS particle having a reporter, where the SERS signature disappears or appears as a function of time. Such time variable particles may be used, for example, to monitor the position of an object moving between several locations, such as a covert taggant added to an object, document, material or substance to verify its receipt at successive locations. In addition, if the surface area of an object, document, material or substance being marked with a SERS particle based taggant is small, it would be advantageous to "erase" the signal associated with a marking particle, and program a new signature as desired or needed for enhanced security.

One embodiment disclosed herein includes particles where the SERS signature is programmed to appear or disappear on demand. For example, an extra level of covert security will result if particles are added to an ink used for authentication or tracking in tax stamps, passports, packaging or similar items, such that the particles can only be spectroscopically detected in conjunction with an external stimulus. Alternatively, particles may be provided that display a distinct signature observable only at predetermined, possibly confidential times.

In an alternative embodiment, the SERS-active signature of a particle can be variable upon selected exposure to an environmental factor, a chemical or other substance. For example, meat or dairy products are typically kept at low temperatures to avoid spoilage. If the SERS signature of a particle associated with these types of product can be programmed to change irreversibly upon exposure to a threshold temperature, e.g. 60 °F, such a particle could be used to track exposure to a threshold condition. A covert taggant can thus be designed both to verify authenticity and monitor product storage and handling. Similarly, a variable SERS-active signature could be used to monitor exposure of a tagged substance to radicals, acid or basic conditions or to many other types of chemical substances or environmental conditions.

In other embodiments, a specific SERS signature may be programmed to appear as a result of a biochemical interaction between two (or more) binding partners in solution. Such systems are typically referred to as proximity assays. One advantage of SERS compared to fluorescence, the typical readout method utilized in a proximity assay, is the relative narrowness of SERS spectral features, generating the possibility of multiplexed assays that can simultaneously track multiple analytes.

The compositions of matter and methods disclosed herein include various types of surface enhanced spectroscopy particles, in particular SERS particles where the spectroscopy signature of the particle changes. The signature or spectroscopic output may selectively be changed, change over time, change in response to an external factor such as an environmental factor or the exposure to a substance or otherwise change in any manner. This class of particles is defined herein as a "programmable SES particle" or (PSP). The PSPs described herein may be tuned to work with SERS, SERRS or other SES techniques.

PSPs as described herein may be readily distinguished from encapsulated SERS nanotags as described in US Patents No. 6,514,767, No. 6,861,263, No. 7,443,489 and elsewhere. SERS nanotags are designed to give a constant, non-varying signal that is immune to environmental, physical, and chemical stimuli, and which does not change over time. In contrast, PSPs are designed to return a controlled but variable signal that can be triggered to change externally or which naturally varies over time.

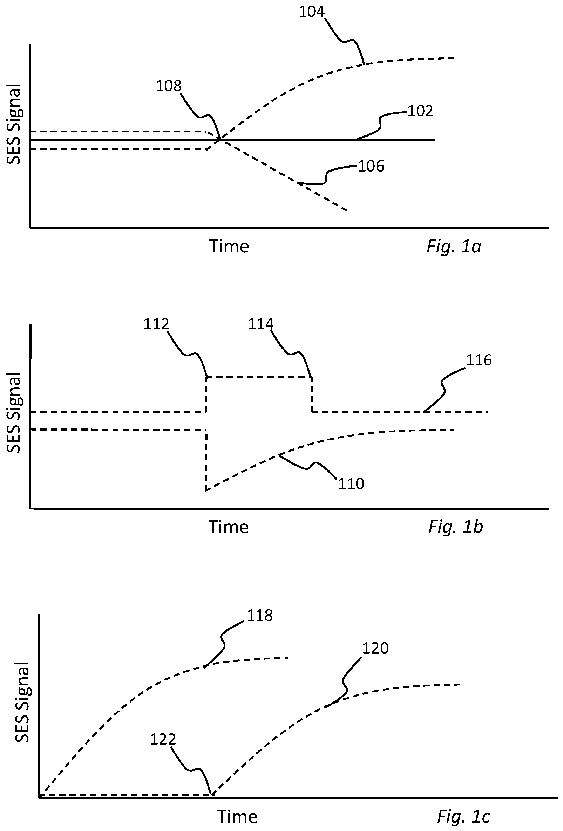

Fig.1a graphically illustrates the functional difference between a known SERS nanotag and a PSP as disclosed herein. The observed signal from a SERS nanotag, trace 102 is a SERS signal that does not vary over time. However, a PSP provides a signal, traces 104 and 106, for example, that varies over time. At a particular time, denoted as the "trigger," point 108 of Fig. 1, the SERS signal obtained from a PSP may begin to change. The change can be an increase or a decrease. The signal change can vary linearly over time, as shown for signal 106 or the signal may vary asymptotically, as shown for signal 104. The change in signal over time can assume an infinite variety of forms. The scope of the present disclosure is not limited to any particular type of signal change. For example, as shown in Fig. 1b, the SERS signal of a PSP can change in response to a trigger and then return to its initial value over time as graphically illustrated with signal trace 110. Alternatively, the SERS signal can change in response to a trigger 112, and achieve a new constant value. Introduction of a second trigger 114 induces a signal change to a second new constant value as shown with signal trace 116. In Fig. 1b, the second constant value is illustrates as equal to the original signal value, but it need not be so.

Fig. 1c illustrates two other non-limiting representative scenarios where the SERS signal obtained from a PSP changes over time. In representative signal trace 118, at time = 0, there is no signal, and a signal grows in intensity over time. In this embodiment, the signal intensity increases over time in the absence of stimulus or trigger. Alternatively the signal could decay over time, with signal decay initiating at time zero. In an alternative scenario as shown on representative signal trace 120, the SERS signal = 0 at time = 0, and remains 0 until a trigger 122 is applied.

Representative triggers can be any number of stimuli, applied either individually or in combination. Examples of triggers include but are not limited to physical, optical, chemical, biochemical, electrical, magnetic, electromagnetic, mechanical, and fluidic or microfluidic phenomena. Other non-limiting examples of triggers include changes in pressure, in volume, in temperature, in mass, in weight, in flow rate, in enthalpy, in entropy, in Gibbs free energy, in the absence or presence or concentration of ions, cations, anions, electrons, atoms, molecules, oxidants, reductants, solvents, molecular complexes, biomolecules, supramolecular species, and/or particles, as well as complexes between or combinations of any of the foregoing. Additional non-limiting triggers include changes in voltage, current, resistance, impedance, redox potential, turbulence, flow rate, porosity, surface area, light (x-ray, deep uv, uv, visible, near-IR, IR, microwave), wavelength of light, intensity of light (for example off-to-on or on-to-off), intensity of sound and frequency of sound. Changes in equilibrium constant can also serve as triggers. In addition, triggers may comprise two or more stimuli, e.g. a change in temperature and pressure, or a change in Gibbs free energy, chemical composition, and redox potential, or a change in flow rate and ionic concentration.

Spatial gradients in any of the above can serve as triggers, especially when coupled to a change in access to the reporter or other SERS-signal generating moiety. For example, a particle may have a gradient in the concentration of reporter molecules on the two sides of the encapsulant. On the inner side (the side facing the SERS-active particle core), there is a high concentration of SERS-signal generating moiety, whereas on the side of the encapsulant facing the external environment, there is no reporter and reporter concentration thus equals 0. This gradient can, under specific circumstances, serve as a trigger by diffusion of the reporter away from the SERS-active particle surface. Such diffusion will not happen in a known SERS nanotag as the result of multiple factors, not least of which are the binding of the reporter molecule to the core surface and the restricted diffusion caused by the encapsulant itself.

However, if a SERS-active particle with a weakly adsorbed reporter is coated deliberately with a layer of porous silica or a layer of silica subsequently made porous, the aforementioned gradient will be sufficient to cause loss of signal starting at time = 0, as shown in Fig. 1c, signal trace 118, but with a SERS signal decrease rather than an increase. Alternatively, a SERS-active particle coated with a nonporous encapsulant (but with no reporter) would show no signal at time = 0, even in the presence of a high concentration of reporter in solution. If a suitable trigger e.g. pH, temperature, or chemical reagent, causes an increase in encapsulant porosity, the reporter in solution will diffuse through the pores in response to the concentration gradient, leading to a growth in SERS signal. This example is depicted in Fig. 1c, signal trace 120.

The time scale over which changes in the SERS signal associated with a PSP can, in many embodiments, be controlled. Signal changes could be caused to occur on timescales as short as that required for obtaining Raman spectra, thus, as short as one or more picoseconds. For example, a photochemical trigger using a pulsed laser could induce a change in SERS signal on such an extremely short timescale. Alternatively, the signal change of a PSP can occur over nanoseconds, microsceconds, seconds, minutes, hours, days, weeks, months, years, or even decades, as a function of the application of a specific trigger or naturally.

The changes in SERS signal, whether appearance or disappearance, does not have to be a complete appearance or complete disappearance. Any observable lesser signal change, for example a 1%, 3%, 5%, 10%, 20%, 30%, 40%. 50%. 60%. 70%, 80% or 90% change in signal intensity will, in many instances, be satisfactory for the purposes described herein. Likewise, the measurement of the change does not have to be absolute; rather, it can be measured as a ratio, or compared to a standard material that does not change SERS signal intensity over time or in response to a selected stimulus. For example, a static SERS particle may be mixed with reporter A and a PSP particle with reporter B. In one example, the static particle is not sensitive or only weakly sensitive to temperature, whereas at temperatures greater than 50 °C, the signal from Reporter A in the PSP decays sharply over time. A comparison of the SERS signal from the PSP and the static particle may provide increased accuracy compared to measuring the PSP output alone.

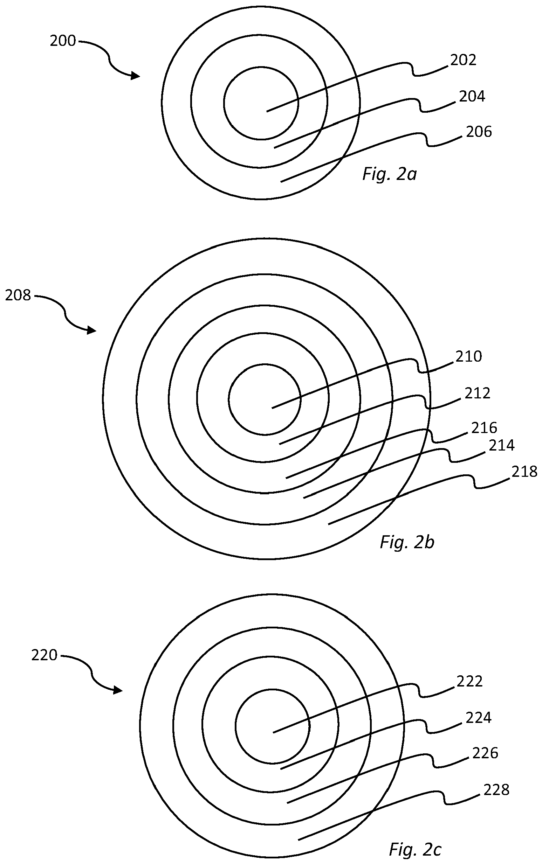

PSPs may be fabricated in a wide variety of geometries. Several non-exclusive, representative geometries are shown in Fig. 2. Fig. 2a schematically illustrates a simple architecture for a programmable SES particle (PSP) 200, which includes a surface enhanced spectroscopy-surface, namely a core 202 that is SERS-active. PSP 200 also includes a programmable reporter, in particular, a layer of reporter molecules 204, and an outer layer 206. The core 202 can be Au, Ag, Cu, Al, Pd, Pt, mixtures of the foregoing, or any other material that exhibits SERS enhancement at any wavelength from 200 nm to 2500 nm, and can have a diameter or effective diameter between 2 nm and 2000 nm. The core can be of any shape or structure. The reporter 204 can be an organic molecule or mixture of molecules, an inorganic molecule or mixture of molecules, a polymer or mixture of polymers, a solid-state material or mixture of solid-state materials, or any combination of the foregoing having a detectable Raman spectrum. The reporter 204 can be, can have or can include a single molecule, multiple molecules, a submonolayer, monolayer, a multilayer, or a film as thick as 1 micron. The outer layer 206 can be silica, titania, zirconia, any oxide or mixture of oxides, a nitride or mixture of nitrides, a chalcogenide or mixture of chalcogenides, a polymer or mixture of polymers, or a combination of any of the foregoing. A feature of the outer layer 206 which distinguishes a PSP from known particles, is that the outer layer is designed to allow or selectively allow access by the external environment to the reporter 204, and vice versa. For example, outer layer 206 could be intrinsically porous, optically porous, or made porous by an external stimuli (e.g. light, heating/cooling, or a chemical reagent). Alternatively, outer layer 206 could be programmed to wholly or partially disappear, either by dissolution, dissociation, or other methods, leaving behind the core 202 and reporter 204.

An alternative PSP composition is shown in Fig. 2b. In this embodiment, the programmable SES particle (PSP) 208 comprises a SERS-active core 210, and a layer of reporter 212. The dimensions for 210 and 212 are as detailed above. Also included in this embodiment is a delivery layer 214, alternatively referred to as a generation layer, a diffusion layer 216 and an outer layer 218. The role of the delivery layer 214 is to harbor and/or generate a material that either increases or decreases the SERS signal. The role of the diffusion layer 216 is to control the amount of time required for the material in delivery/generation layer 214 to reach the core 210 and/or the reporter 212.

Another alternative non-limiting geometry for a PSP is shown in Fig. 2c. In this embodiment, PSP 220 comprises a core 222, with shells 224, 226, and 228 respectively. In one specific implementation, the SERS-active surface may be shell 224, which can be between 1 and 250 nm in thickness, the programmable reporter layer may be shell 226, which can be between 0.5 nm and 500 nm in thickness, and the encapsulant may be shell 228, which can be between 1 nm and 250 nm in thickness. In this specific embodiment, core 222 is a delivery layer between 2 and 500 nm in diameter that can be triggered to alter the SERS activity of the shell 224. For example, core 222 could be a metal, which using heat as a trigger, will diffuse into the SERS-active layer 224. If layer 224 is Au and core 222 is Ag, two metals that freely interdiffuse in a temperature-dependent fashion, the resulting alloy could exhibit greater or less SERS-activity depending on the excitation wavelength used and the relative dimensions of core 222 and shell layer 224.

Alternatively, with respect to the general configuration of the PSP of Fig. 2c, core 222 could be a SERS-active surface, shell 224 could be a programmable reporter as defined above, shell 226 could be a diffusion layer, and shell 228 could be a generation layer.

It is important to note that the examples schematically illustrated in Fig. 2 are illustrated with single, spherically symmetric particle geometries, but the concepts and methods disclosed herein apply equally well to all shapes of particles, including but not limited to prisms, rods, triangles, tetrahedra, octrahedra, egg-shaped, cones, trapezoids, pyramids, stars, hollow particles of any of the foregoing, and any other shape or combination of shapes that can be described using Euclidean geometry. In addition, particle shapes that cannot be described simply using Euclidean geometry, or which possess no symmetry, e.g. a "swiss cheese" particle made by removing the Ag from an Ag/Au alloy, or a particle in the shape of a crumpled piece of paper, made by unpeeling of a very thin metal film, are also included.

Likewise, while the examples in Fig. 2 are illustrated with a single particle, it is to be understood that two or more particles in aggregates or other groupings are within the scope of the embodiments disclosed herein, including but not limited to aggregates of 2, 3, 4, 5, 6, 7, 8, 9, 10, 12, 15, 20, 25, 30, 35, 40, 45, 50, 55 or more particles.

It is also important to note that the modulation or change of the SERS signal obtained from a PSP, whether an increase or decrease, or an appearance or disappearance, can occur at the level of the SERS-active material, the reporter, the encapsulant and/or barrier layer, or any combinations thereof. Thus, the signal of a PSP may be "turned on" or "turned off" or "turned up" or "turned down" by altering the SERS-active material, by altering the reporter, by altering the encapsulant/barrier layer, or combinations thereof. Such alterations might include conversion to different compositions, but conversion is not always necessary. For example, the SERS signal of a PSP particle aggregate may be modulated by adjusting the spacing between particles. Likewise, the SERS signal of a PSP can be modulated by changing the orientation of a reporter with respect to the SERS-active surface. Alternatively, the physical, optical or chemical porosity of a barrier layer can be increased or decreased. In selected examples, the composition of the PSP does not change, but the geometry and/or structure of the PSP is altered. In view of the foregoing, several specific but non-limiting examples of methods, reactions or processes which may be exploited to change the signal output of a PSP may be described.

### Triggering a PSP through Action upon a Delivery Layer

Although modulation or change of the PSP signal can occur through mechanisms working on any layer or the particle core, specific embodiments are described in detail below which primarily function though actions on a delivery layer, for example layer 214 of fig. 2b or layer 226 of Fig. 2c. Other methods involve action upon the encapsulant or outer layer, for example elements 206, 218 or 228 of Fig. 2a-c, or the corresponding encapsulant/outer layer of a particle having a non-spherical shape.

The following discussion will center upon a particle such as PSP 208 of Fig. 2b for the purposes of illustrating concepts relevant to all particle geometries or shapes. As described above with respect to Fig. 2b, one fabrication strategy is to surround the core/reporter combination with three conformal layers, an inner diffusion layer 216 which encapsulates the core and reporter, 210, 212 which is in turn encapsulated by a delivery layer 214, also called a "generation layer", which is in turn encapsulated by the outer layer 218 which may be an encapsulant. This structure may be referred to as a "shell/shell/shell" structure, with the understanding that for the following examples, the middle shell is the delivery layer or generation layer 214.

An external stimulus, for example heat or light, or a chemical stimulus may be used to cause the production of a reagent in the delivery layer that will degrade the SERS signal by action on the reporter, or the surface layer of atoms of the core. Representative delivery layer reagents may include but are not limited to a strong acid, strong base, cyanide (-CN), or a highly-reactive C- or O-based free radicals (e.g. RO•). The reagent diffuses from the delivery layer to the core/reporter surface and degrades the SERS signal. In the case of a free radical, for example, the degradation mechanism is oxidation of the sub-monolayer of reporter, while in the case of cyanide; it is the etching of the first layer of metal atoms.

The time required for degradation is based on three variables that can be completely or partially controlled via the design of the particle and trigger system: the amount of stimulus reaching the delivery layer, which is a function of the composition of the outer layer; the amount of reagent produced, which is a function of the thickness of and chemistry employed in the delivery layer; and the amount of reagent reaching the core surface, which is a function of the thickness and porosity of the diffusion layer.

Heat may be used as a trigger to increase, decrease, turn on or turn off the signal obtained from a PSP. Any substance that releases or forms an active reagent in response to temperature as described above could be utilized in a heat activated delivery layer. For example, radicals of poly(*N*-vinylcarbazole) can be generated in a delivery layer by the thermal decomposition of peroxides. Alternatively, the heat induced fresh fracture of silica leads to the generation Si-O radicals. Alternatively, pure diphenyliodonium tetrafluoroborate and diphenyliodonium hexafluorophosphate have been found to generate hydrogen fluoride by pyrolysis at 239°C and at 150°C in the presence of anisole or nitrobenzene. In addition, *o*-nitrobenzyl tosylate acts as a thermal source of *p-*toluenesulfonic acid at temperatures between 100 and 110°C. In addition, thermal decomposition of phthalamic acid derivatives chemically bonded to the surface of silica can be used to generate mixtures of ammonia, methylamine, diethylamine and triethylamine. The foregoing and similar processes can be incorporated into a delivery layer as described above.

In addition, certain polymers depolymerize when they are subjected to temperatures above their corresponding ceiling temperatures. The process starts by bond scission in the polymer backbone, creating a pair of radicals which trigger the sequential depolymerization of the monomers (unzipping). If the temperature is considerably higher than the ceiling temperature of the polymer, many bonds will break and a large number of small radicals will form in a polymer delivery layer. Some or many of those radicals may penetrate the inner or diffusion layer and reach the core surface, destroying the radical sensitive reporter molecule and rendering the PSP inactive. Typical examples are poly α-methylstyrene (ceiling temperature = 61 °C) and polymethylmethacrylate (ceiling temperature = 230-300 °C). The depolymerization temperature can be modified by adding co-monomers to the polymer chain or by choosing polymer end groups that slow down the process.

Any number of photochemical processes my by utilized or exploited to trigger the generation of a reagent in a delivery layer as described above. The following is a non-exclusive discussion of various photochemical triggering possibilities. For example, photoacid generators are commonly used in chemically amplified resists (CAR), in which strong acids are generated upon irradiation of the photoacid. Although the small molecule photoacid is typically blended with a polymer for the final application, it is possible to incorporate a photoacid group into a polymer chain. Different polymeric chains containing pendant photoacid groups (i.e. phenydimethylsulfonium triflate groups) may be synthesized to build the delivery layer of a shell/shell/shell PSP particle. In selected embodiments, irradiation of the terminated tag will liberate a strong acid. The acid can penetrate the diffusion layer of the tag and will react with an acid sensitive reporter, eliminating the SERS signal from the PSP.

Triphenylmethane leucohydroxide derivatives liberate hydroxide ions upon UV irradiation. Likewise, alkoxy derivatives of triphenylmethane can generate alkoxide ions in the same manner. These photosensitive compounds can be built into polymers to yield photosensitive, base releasing materials. These and similar materials may be synthesized and used to build the delivery layer of a triple shell PSP particle. UV irradiation of the particle will liberate hydroxide and/or alkoxide ions, which will penetrate the diffusion layer of the PSP particle and will react with a base sensitive reporter, rendering the particle inactive.

Polymers containing benzoyn ether derivatives fragment upon irradiation to form benzoyl and α-alkoxybenzyl primary radicals. Polymers containing pendant benzoyn ether groups may be synthesized to build a delivery layer of a shell/shell/shell PSP particle. Irradiation of the particle will liberate radicals that will penetrate the inner layer of the PSP and will react with the radical sensitive reporter, rendering the particle inactive. A generic structures of a photosensitive polymer that generate a) acid, b) base and c) free radicals are:

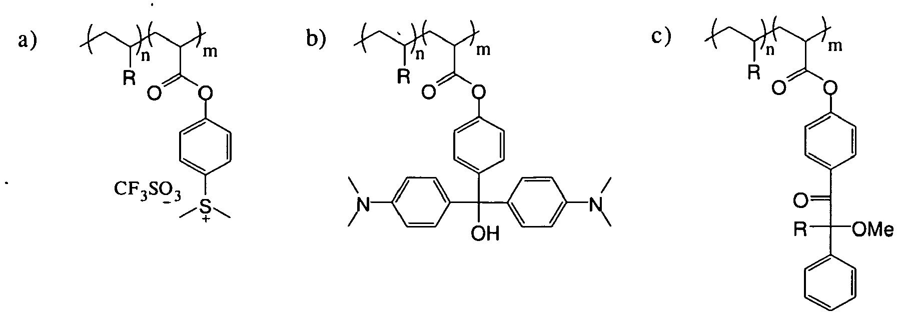

Many other chemicals or materials can be used to synthesize the delivery layer of a PSP. For example, a polymeric photobase generator containing oxime-urethane groups can be prepared by copolymerization of methyl methacrylate and methacryloxyethyl benzophenoneoxime urethane. Alpha-keto carbamates could also be useful as photoprecursors of amines. These materials can undergo light triggered photocleavage both in the solid-state and in solution to give free amines. The photoactive benzoinyloxycarbonyl groups of these compounds are active with ultraviolet radiation below 400 nm. Photoactive 2-nitrobenzylcarbamates may also be used for this purpose.

The Uv-based photogeneration of free radicals is widely used in the curing of polymers, and a variety of initiators are known. Two families of free radical initiators are well known: "hydrogen abstraction type" or "alpha cleavage type." Examples of photoinitiators which could be used in a delivery layer and which are activated in the ultraviolet and visible range include Ciba® IRGACURE® and Ciba® DAROCUR®.

Another example of a radical initiator that can be both thermally or photochemically initiated is benzoyl peroxide. Alternatively, one can use Azobisisobutyronitrile (AIBN) as a radical initiator. The most common chemical reaction of AIBN is one of decomposition, eliminating a molecule of nitrogen gas to form two 2-cyanoprop-2-yl radicals, as shown below:

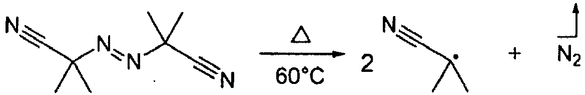

### Triggering a PSP through Action upon an Encapsulant

The foregoing discussion detailed various strategies for triggering a PSP by action upon a delivery layer. Alternative strategies include action upon an encapsulant or outer layer. In the absence of encapsulants, SERS active cores with adsorbed reporter molecules are generally unstable. Eventually, the SERS signal disappears by adsorption of impurities/desorption of reporter, fouling of the SERS surface, and/or diffusion of reporters away from EM hot spots. Thus, a viable approach to PSP signal change is to trigger removal of the encapsulant or trigger the permeability of the encapsulant, thereby initiating signal reduction or signal increase, depending upon the system used. There exist many mechanisms which could be used to directly act upon an encapsulant layer. A non-exclusive sampling of possible mechanisms or methods is discussed below.

The polymer ceiling temperature phenomena may be used to trigger the degradation of a polymeric encapsulant. When a polymer is heated above its ceiling temperature, bond scission takes place in the polymer backbone, triggering the sequential depolymerization of the monomers (unzipping). Representative examples of polymers with low ceiling temperatures are poly α-methylstyrene (ceiling temperature = 61 °C) and polymethylmethacrylate (ceiling temperature = 230-300 °C). The depolymerization temperature can be modified by adding co-monomers to the polymer chain or by choosing polymer end groups that slow down the process.

Thermally depolymerizable tertiary polycarbonates degrade when heated at around 200 °C (the actual degradation temperature depends on polymer structure), resulting in only volatile compounds as end products, thus leaving no solid residue. The degradation temperature is lowered by acid catalysis. Poly(olefin sulfone)s also undergo unzipping when heated in the presence of amines. The amino groups can be foreign, or they can be photogenerated from pending photobase generating groups in the same polymer.

The environmental degradation of encapsulant layers can also be impacted by the thermal-mechanical manipulation of thermally responsive polymers. For example, poly(N-isopropylacrylamide), or pNIPAM, is well known to undergo thermally-induced contraction. At low temperatures, the pNIPAM hydrogel is swollen with water, but collapses into a hydrophobic, globular state upon reaching its lower critical solution temperature (LCST). Conveniently, the LCST can be tuned by the incorporation of copolymers, and tends to be in environmentally relevant ranges centered near 30°C. This swelling/de-swelling behavior can be used to impact the encapsulant porosity and even to shuttle environmental "contaminants" to the core, thus poisoning the core and/or displacing the original reporter. Another possible application for these materials is to put them directly on the core-reporter interface, and use thermally induced contraction to force the reporter molecules off of the core surface.

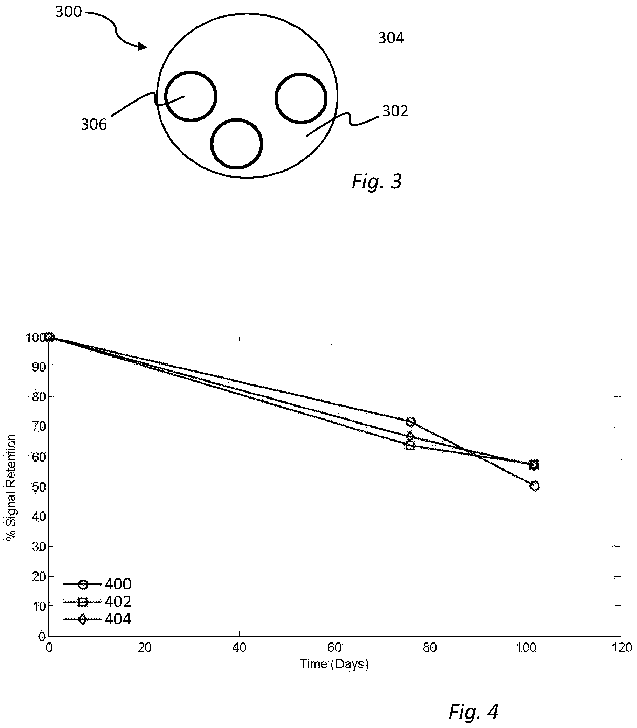

An alternative approach to encapsulant removal is to make the encapsulant intrinsically unstable, or at least weakly stable. One approach to this is shown in Fig 3, through the use of organic-soluble reverse micelles 300. The micelles are non-covalent assemblies of molecules with both hydrophobic and hydrophilic functional groups, such that an aqueous compartment 302 can be contained in a non-polar phase 304. Techniques for the formation of reverse micelles are well-understood, as are methods by which particles can be incorporated into them. In this case, naked core and reporter molecules 306 may be included. In non-polar solvents, the particles (for simplicity shown as solid spheres) may freely separate, and as a result there is no plasmon intensity at selected interrogation wavelengths. Under heat and/or pressure, the reverse micelle ruptures, driving particle aggregation and reporter adsorption. Upon rupture, a SERS signal may be observed. Over time, The SERS signal degrades as other materials adsorb to and/or foul the unprotected SERS-active surface.

A wide variety of natural, synthetic, and biosynthetic polymers can be bio-degraded by the environment or by bacteria. One approach to encapsulant triggering is to incorporate hydrolytically (water)-reactive chemical linkages such as anhydrides, or to a lesser extent, amides or esters into a polymer encapsulant. For the latter, catalytic degradation by enzymatic cleavage is preferred. Biodegradable materials containing hyrdolyzable linkages are widely available, including those based on polycaprolactone, polylactide, and polyglycolide. Environmentally degradable materials include polysaccharides (e.g. starch) and poly(hydroxyalkanoates). One approach is thus to coat a core/reporter combinations with such polymers, and let natural biodegradation occur over time. An advantage to this approach is its simplicity; also a longer degradation time is likely.

Selected PSP triggering approaches described above involve the triggered disruption or degradation of an encapsulant layer. An alternative approach is to engineer encapsulants with built-in pores, such that environmental factors or specific chemical factors can directly access the reporter/core surface. Alternative methods and materials may be selected to tune the time period required to change the signal obtained from a PSP.

For example, aerogels exhibit the lowest density of any known solid, and accordingly have extraordinary effectiveness as a thermal insulator. Aerogels are usually composed of SiO₂, and are derived from silica gel through supercritical drying. Encapsulants can be made out of silica gel, and then converted into aerogels. Such PSP particles by themselves should be much more thermally stable that those fabricated with conventional silica shells, and accordingly represent an alternative approach to making thermally stable particles. Aerogels however, exhibit very high porosity, meaning that external species can freely diffuse in to the core/reporter interface.

Another approach to controlled degradation includes encapsulating particles with polymers that are permselective, allowing only certain gases to reach the particle surface. This is a useful method insofar as the dissolved gas content in liquids can be made low, so particle shelf life is not impacted, but then in the presence of O₂, ozone, or other reactive gas, degradation can be made to occur by reaction of the gas with the reporter. A well-studied class of materials is polymers that are oxygen-permeable, for example, the material used in modern contact lenses. Examples of polymers useful for this purpose include but are not limited to poly(dimethylsiloxane), polystyrene, poly(vinylchoride), polymethyl(methacrylate), a variety of fluoropolymers, and a variety of cellulose derivatives. These polymers and derivatives thereof can be used directly as encapsulants. Alternatively, they can be placed on top of porous SiO₂, which would be permeable to all gases.

In other embodiments, PSPs serve as tags for labeling objects or materials, e.g., for anti-counterfeiting or authentication purposes, or for encoding the history of an object moving through a manufacturing process or supply chain. The ability of a PSP to be programmed as described herein enhances the usefulness of a PSP as a taggant. In these applications, one or more PSPs are associated with an object or material and later "read" by an appropriate spectroscopy method to determine the identity of the particle or particles and obtain information about the tagged object. The acquired spectrum can be compared to a reference spectrum or to a spectrum of the particles acquired before they were associated with the object. If necessary, suitable corrections can be made to account for background emission from the object. Authentication can occur at any desired point during the lifetime of the object, e.g., upon receipt of a manufactured object by a retailer or upon sale of an antique object.

Each PSP or group of PSPs, with its unique and possibly variable spectrum, corresponds to or represents a particular piece of information. Any type of information can be represented by a PSP, depending upon the application. For example, a PSP or group of PSPs can represent an individual object such as an item of sports memorabilia, a work of art, an automobile, or the item's owner or manufacturer; a class of objects, such as a particular formulation of pharmaceutical product; or a step of a manufacturing process. The information represented by a particular spectrum or PSP type can be stored in a database, computer file, paper record, or other desired format.

The small, robust, non-toxic, and easily-attachable nature of PSPs allows their use for tagging virtually any desired object. The tracked object can be made of solid, liquid, or gas phase material or any combination of phases. The material can be a discrete solid object, such as a container, pill, or piece of jewelry, or a continuous or granular material, such as paint, ink, fuel, or extended piece of, e.g., textile, paper, or plastic, in which case the particles are typically distributed throughout the material.

Examples of specific materials or objects that can be tagged with PSPs, or into which PSPs can be incorporated include, but are not limited to:
- Packaging, including adhesives, paper, plastics, labels, and seals
- Agrochemicals, seeds, and crops
- Artwork
- Computer chips
- Cosmetics and perfumes
- Compact disks (CDs), digital video disks (DVDs), and videotapes
- Documents, money, and other paper products (e.g., labels, passports, stock certificates)
- Inks, paints, varnishes, lacquers, overcoats, topcoats, and dyes
- Electronic devices
- Explosives and weapons
- Food and beverages, tobacco
- Textiles, clothing, footwear, designer products, and apparel labels
- Polymers
- Insects, birds, reptiles, and mammals
- Powders
- Luxury goods
- Other anti-counterfeiting substances or materials, such as holograms, optically variable devices, color-shifting inks, threads, and optically-active particles
- Hazardous waste
- Movie props and memorabilia, sports memorabilia and apparel
- Manufacturing parts, automobile parts, aircraft parts, truck parts
- Petroleum, fuel, lubricants, gasoline, crude oil, diesel fuel, fuel additive packages, crude oil
- Pharmaceuticals, prescription drugs, over-the-counter medicines, and vaccines

PSPs can be associated with the material in any way that maintains their association, at least until the particles are read. Depending upon the material to be tagged, the particles can be incorporated during production or associated with a finished product. Because they are so small, the particles are unlikely to have a detrimental effect on either the manufacturing process or the finished product. The particles can be associated with or attached to the material via any chemical or physical means that does not inherently interfere with particle functionality. For example, particles can be mixed with and distributed throughout a liquid-based substance such as paint, oil, or ink and then applied to a surface. They can be wound within fibers of a textile, paper, or other fibrous or woven product, or trapped between layers of a multi-layer label. The particles can be incorporated during production of a • polymeric or slurried material and bound during polymerization or drying of the material. Additionally, the surfaces of the particles can be chemically derivatized with functional groups of any desired characteristic, for covalent or non-covalent attachment to the material. When the particles are applied to a finished product, they can be applied manually by, e.g., a pipette, or automatically by a pipette, spray nozzle, or the like. Particles can be applied in solution in a suitable solvent (e.g., ethanol), which then evaporates.

PSPs have a number of inherent properties that are advantageous for tagging and tracking applications. They offer a very large number of possible codes. The codes may be variable over time. For example, if a panel of PSPs is constructed with 20 distinguishable Raman spectra, and an object is labeled with two PSPs, there are 20*19/2 = 190 different codes. If the number of particles per object is increased to 5, there are 15,504 possible codes. Ten particles per object yields 1.1 x 10⁶ different codes. A more sophisticated monochromator increases the number of distinguishable spectra to, e.g., 50, greatly increasing the number of possible codes. Alternatively, different amounts of PSPs can be used to generate an exponentially-increased number of possible codes. For example, with just four different particle types (N=4), present at three different intensity levels (e.g. High, Medium, Low) (L=3), chosen three at a time (P =3), can generate 58 different codes. With N=10, P=3, L =1, the number of codes is 175. With N=50, P=5, L=4, over a billion codes are possible.

In some embodiments, the PSP may be applied to a document or other item in an ink or other marking material. Inks include, but are not limited to flexographic ink, lithographic ink, silkscreen ink, gravure ink, bleeding ink, coin reactive ink, erasable ink, pen reactive ink, heat reactive ink, visible infrared ink, optically variable ink, and penetrating ink. photochromic ink, solvent/chemical reactive ink, thermochromic ink, and water fugitive ink. A PSP may also be applied in electrophotographic and ink jet printing machines and other systems including offset lithography, letterpress, gravure, heliogravure, xerography, photography, silkscreening systems, systems for imagewise deposition of discrete quantities of a marking material on a substrate surface, such as paint, chemical, and film deposition systems; and systems for integration of colorant materials in an exposed surface of a fibrous substrate, such as textile printing systems.

It should be noted that additional security features may be included or utilized along with PSP tags for a particular item or documents. One such additional security feature may be a separate security ink, such as bleeding ink, coin reactive ink, erasable ink, pen reactive ink, heat reactive ink, visible infrared ink, optically variable ink, penetrating ink. photochromic ink, solvent/chemical reactive ink, thermochromic ink or water fugitive ink. The PSP tags may be applied as part of the ink, or in a separate step. Other non-ink based security features which may be utilized in addition to PSP tags for document or item marking include the use of an ascending serial number (horizontal and/or vertical format), bar code and numerals, colored fibers, embedded security thread, face-back optical registration design (transparent register), foil imprints, holograms, latent impressions, micro printing, optical variable devices (OVD), planchettes, raised marks, segmented security threads, and watermarks.

PSP security tags may be applied by coating an image, including but not limited to a hologram image, made with toner or ink compositions known in the art, as with an overcoat varnish, or a starch overcoat.

In the case of documents with other security features, such as those including polymer threads or metal foils, the PSP may be applied to additional feature, such as the thread or the foil. Single PSP tags may be considered to represent a bit of data that may be changeable according to the methods described herein. Thus groups of distinguishable PSPs may be applied to constitute an "alphabet" and combined as words or encoded information, which may be selectively variable, or variable over time.

PSPs can be identified using a conventional spectrometer, for example a Raman spectrometer. In fact, one benefit of using SERS PSPs is the versatility of excitation sources and detection instrumentation that can be employed for Raman spectroscopy. Visible or near-IR lasers of varying sizes and configurations can be used to generate Raman spectra. Portable, handheld, and briefcase-sized instruments are commonplace. At the same time, more sophisticated monochromators with greater spectral resolving power allow an increase in the number of unique taggants that can be employed within a given spectral region. For example, the capability to distinguish between two Raman peaks whose maxima differ by only 3 cm⁻¹ is routine.

Typically, if a suitable waveguide (e.g., optical fiber) is provided for transmitting light to and from the object, the excitation source and detector can be physically remote from the object being verified. This allows PSPs to be used in locations in which it is difficult to place conventional light sources or detectors. The nature of Raman scattering and laser-based monochromatic excitation is such that it is not necessary to place the excitation source in close proximity to the Raman-active species. Moreover, PSPs are amenable for use with all known forms of Raman spectrometers, including some more recent implementations, including spatially offset Raman, Raman absorption spectrometers, instruments to measure Raman optical activity, and so forth.

Another characteristic of PSPs is that the measurement of their spectra does not need to be strictly confined to "line of sight" detection, as with, e.g., fluorescent tags. Thus their spectrum can be acquired without removing the particles from the tagged object, provided that the material is partially transparent to both the excitation wavelength and the Raman photon. For example, water has negligible Raman activity and does not absorb visible radiation, allowing PSPs in water to be detected. PSPs can also be detected when embedded in, e.g., clear plastic, paper, or certain inks.

PSPs also allow for quantitative verification, because the signal intensity is an approximately linear function of the number of analyte molecules. For standardized particles (uniform analyte distribution), the measured signal intensity reflects the number or density of particles. If the particles are added at a known concentration, the measured signal intensity can be used to detect undesired dilution of liquid or granular materials.

### EXAMPLES

The following examples are provided for illustrative purposes only and are not intended to limit the scope of the invention.

### Example 1

Certain SERS-active molecules can be incorporated into a PSP that will exhibit a SERS signal that decays over time. For example, Fig. 4 graphically illustrates the SERS signal behavior of three samples of PSPs, represented by data sets 400, 402 and 404 respectively. The PSPs of example 1 have geometry 200 (Fig. 2a) made using a core 202 of 60-nm diameter Au colloid, a reporter layer 204 of a submonolayer of 2-quinolinethiol, and an outer layer 206 of porous silica. The y-axis in the graph shows the SERS intensity of the sample PSPs presented as a ratio against a standard to eliminate instrument drift over time. The x-axis shows time, and the graph illustrates a consistent decrease in SERS signal over the course of 3 months for these PSPs stored in water at ambient temperature. Over time, the SERS spectra of these PSPs did not change in terms of peak location or ratios; rather, there is a simple decrease in intensity. Since the Au core does not change over time, the signal loss is due to diffusion of the reporter through the outer layer. Fig. 4 thus illustrates the signal obtained from a PSP having a signal level that changes over time without a trigger.

### Example 2

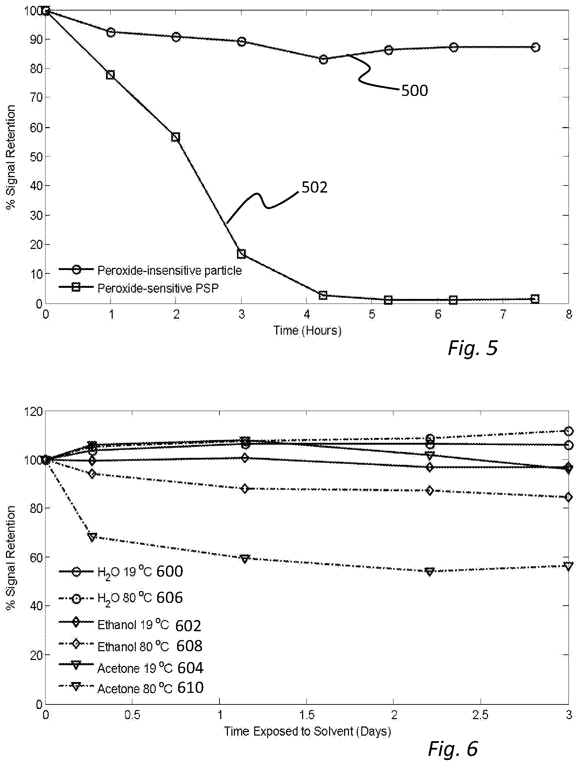

Example 2 features a particle of a geometry 220 (Fig. 2c) with a core 222 of 90-nm diameter Au, a reporter layer 224 consisting of a submonolayer of trans 1,2-bis(4-pyridyl)ethylene (BPE), a 20-nm thick layer permeable silica diffusion layer 226, and an organic barrier layer 228. The addition of the organic barrier layer prevents peroxide from reaching the surface and oxidizing the reporter. Fig. 5 graphically illustrates the signal strength of a PSP particle with an organic barrier (data set 500) versus one without an organic barrier (data set 502). If the organic barrier layer is removed, or if the barrier layer is not added (to yield a particle of geometry 200), the SERS signal decays over the course of a few hours upon the addition of peroxide.

### Example 3

The trigger utilized for a particular PSP can be a combination of factors. Fig. 6 graphically illustrates the SERS response a particle of geometry 200 with a porous silica outer layer 206, a 90-nm diameter Au core 202 and BPE as a reporter 204 after multi-day exposure to organic solvents and/or heat. Exposure to water (Fig. 6, data set 600,) acetone (data set 602), or ethanol (data set 604) does not change the SERS signal over time, and heating to 80 °C in water (data set 606) also has no impact. However, heating the PSP in ethanol and acetone (data points 608, 610) leads to a noticeable loss in SERS signal.

### Example 4

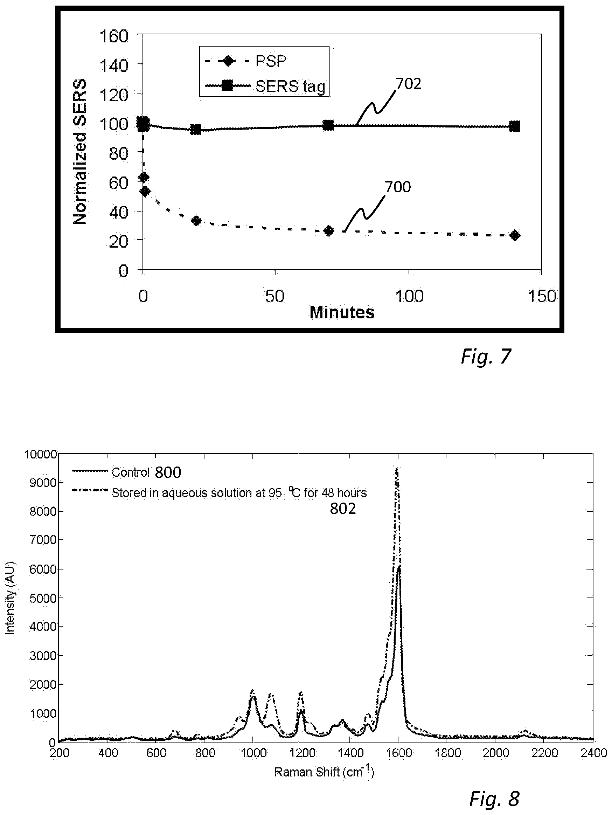

Fig. 7 illustrates the rapid chemical de-activation of a PSP having a specific shell. A silica shelled PSP of geometry 200, when treated with a small amount of Ag⁺, is rapidly deactivated as shown in data set 700. In comparison, a PSP with a polymer shell shows no change in signal upon exposure to Ag⁺ (data set 702).

### Example 5

Fig. 8 illustrates that the SERS signal from a PSP can be increased in response to a stimulus. Using a particle of geometry 200, with a core of 90-nm diameter Au, a 20-nm outer layer 206, and a reporter layer comprising 5-(4-pyridyl)-1,3,4-oxadiazole-2-thiol (POT), one sample (data set 800) was kept at room temperature in aqueous solution, while the other sample (data set 802) was kept at 95 °C in aqueous solution for 48 hrs. In response to this heating, a nearly 50% increase in signal strength was observed.

### Example 6

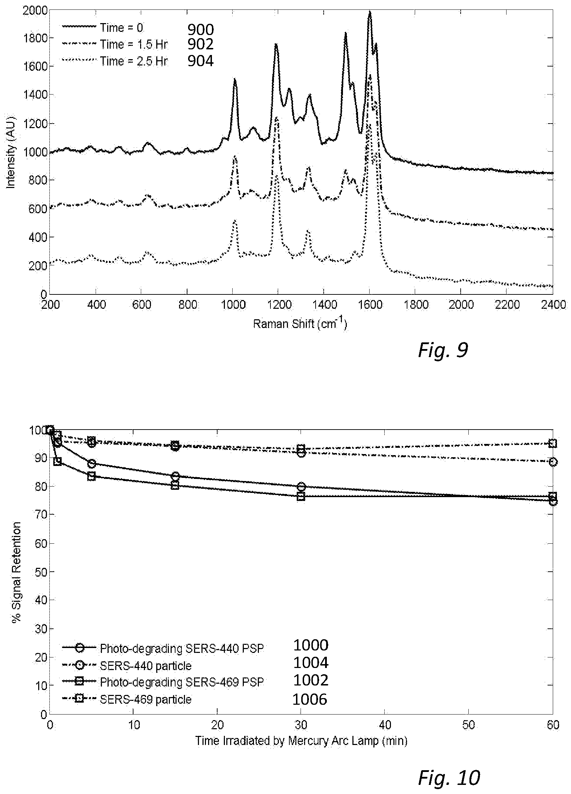

Fig. 9 illustrates that the SERS signal of a PSP can be reduced through selection of the reporter layer molecule and an appropriate stimulus. A particle geometry of 200, with a core 202 of 90-nm diameter Au, and a 20-nm outer layer 206 was prepared with a reporter layer 204 of 5-(5-nitro-2-furyl)-1,3,4-thiadiazole-2-thiol (NFTT) to produce a PSP. The same core particle geometry was used with a reporter layer 204 of BPE to produce a temperature insensitive standard particle. The PSP and standard particle were together placed in a varnish and deposited on a paper substrate as a thin film. As the film was exposed to ambient air at 105 °C the SERS signal derived from the PSP (with a salient peak at 1500 wavenumber) decrease over time (data sets 900, 902 and 904 taken at 0, 1.5 and 2.5 hours respectively), while the SERS signal derived from the temperature insensitive particle remained stable.

### Example 7

Fig. 10 illustrates the signal obtained from a PSP of geometry 220, where 222 is a SERS active core, 224 is a reporter layer, 226 is a generation layer containing "hydrogen abstraction type" functional groups and 228 is a protective layer. Irradiating the PSP with light from a 100 W mercury arc lamp caused radicals to form, attack reporter molecules, and resulted in a decrease in SERS signal as shown in data sets 1000 and 1002. Corresponding particles of geometry 200, not having a generation or delivery layer showed relatively negligible change when irradiated (data sets 1004 and 1006.) The particles which produced data sets 1000 and 1004 included a reporter layer of BPE, whereas the particles that produced data sets 1002 and 1006 included a reporter layer of (2-(4-Pyridyl)-2-cyano-1-(4-ethynylphenyl)ethylene).

### Example 8

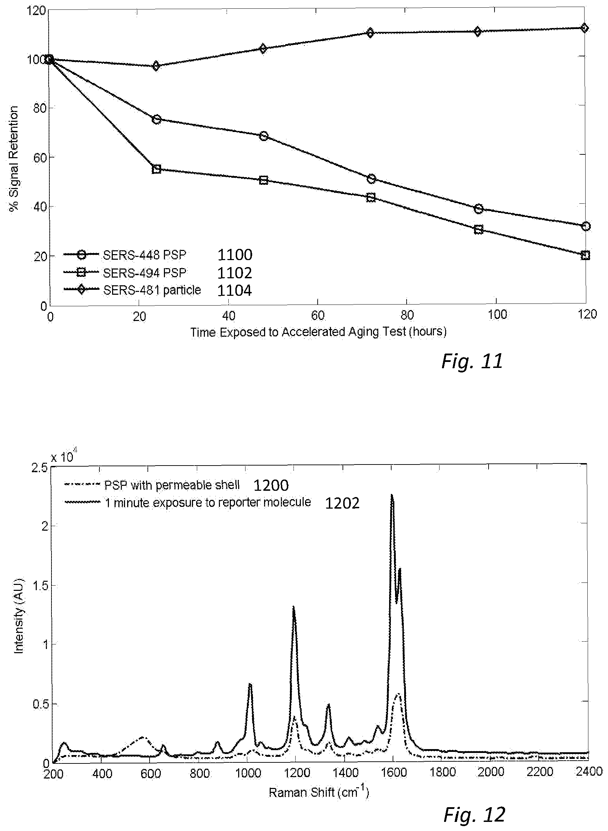

Fig. 11 illustrates the signal reduction of selected PSPs triggered by a combination of environmental stimuli. The PSPs of this example were placed in an environment including the exposure to irradiation from a xenon arc lamp generating 0.59 W/m² at 340 nm, a relative humidity of 30%, and an ambient air temperature of 35 °C. A black body temperature sensor in this environment measured 63 °C. PSPs having a 2,4-Diamino-6-(2-(4-pyridyl)ethen-1-yl)1,3,5-triazine reporter ("SERS-448",data set 1100) or a 4-Pyridinealdazine reporter ("SERS-494", data set 1102) are shown to decrease in signal while a non reactive particle having a Azobis(pyridine) reporter ("SERS-481", data set 1104) under the same stimuli was unaffected.

### Example 9

Fig. 12 illustrates the rapid activation of a PSP. Particles having geometry 200 with a porous silica outer layer 206, but no reporter layer, shows little initial SERS signal (data set 1200). Upon 1 minute of exposure of the particles to a solution containing a reporter, in particular BPE.

### Example 10

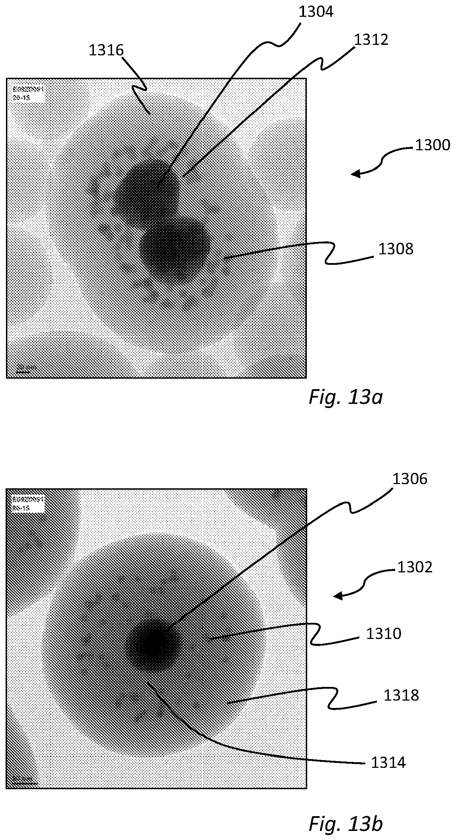

Fig. 13a and 13b shows transmission electron microscope (TEM) images of two similar core/reporter/shell/shell/shell structures 1300 and 1302 respectively. The cores 1304 and 1306 are of identical dimensions in both particle constructions, and are a 90-nm diameter solid Au sphere. In both cases, the middle shell 1308 and 1310 is a mock delivery layer consisting of a monolayer of 12-nm diameter Au particles. The inner shells 1312 and 1314 and outer shells 1316 and 1318 are of silica. In particle 1300 the 12-nm diameter mock delivery layer Au particles 1308 are positioned 20 nm away from the core surface, while in the other particle, 1302 the mock delivery layer Au particles 1310 are positioned 60 nm away. It is important to note that the TEM images of Figs. 13 a-b show a structure having three dimensions in a two dimensional format. Thus, certain 12-nm particles which define the mock delivery layer appear to be close to or on the core. These apparently "close in" particles are actually particles that are relatively higher or lower with respect to the two dimensional plane of the illustration and thus are actually spaced 20 or 60 nm from the core, but placed above or below the core in the substantially spherical delivery layer. The PSPs of Example 10 were prepared as follows:

### 1. Preparation of inner shell.

SERS tags prepared by the methods of US Patents No. 6,514,767, No. 6,861,263, No. 7,443,489 were used as a starting material. The original tags have a glass coating of approximately 25 nm thickness. A thicker glass shell was grown by mixing 100 µL of initial tag (at a concentration of ∼ 7 x 10(14) particles/L) with 400 µL of ethanol, 25 µL of concentrated NH₄OH and 10 µL of TEOS. After an hour, the λₘₐₓ of the extinction spectrum had shifted by approximately 24 nm, indicating a significant thickening of the silica shell. Thus, the reaction was terminated and tags purified by centrifugation and resuspension into ultrapure water.

### 2. Application of delivery layer.

In order to promote adsorption of colloidal gold to the surface of the nanotags, they were first coated with polyallylamine hydrochloride (PAH), a positively charged polymer. 1 mL of water and 100 µL of 100 mg/mL aqueous PAH solution was placed in a microcentrifuge tube. For both thin and thick SiO₂-coated tags, 20 µL of tags (3.5 x 10(14) particles/L) were rapidly added to the diluted PAH, followed by approximately 1 hour on the rotator. These were purified by 4 rounds of centrifugation, resuspending each time in water.

Twenty microliters of the tags (at ∼30x) were then added to 1.5 mL of 12 nm Au colloid [Grabar reference]. These were put on the rotator for 3 hours, after which there was no evidence of aggregation. The 'reaction' was terminated by adding 50 µL of 1x PIMA, which was given 30 minutes to adsorb fully to the tags. Again, centrifugation was used (3 rounds, resuspend water each time) to clean Au-studded nanotags from free colloidal Au. After the last spin, tags were resuspended in 250 µL of water.

### 3. Exterior Shell Growth

In order to grow an exterior glass shell, 1 mL ethanol, 75 µL of NH₄OH and 5 µL of TEOS were added. After 1 hour on the rotator, it was obvious that free SiO₂ was being formed, so samples were centrifuged to stop the reaction and clean the samples.

TEM images were acquired that confirmed the desired core-shell/shell/shell architecture.

## Claims

1. A programmable SES particle comprising:
a surface-enhanced spectroscopy (SES)-active surface; and
a programmable reporter associated with the SES surface;
wherein the programmable reporter comprises:
a SES-active reporter associated with the SES-active surface,
a delivery layer associated with at least one of the SES-active reporter and the SES-active surface; and
an outer shell surrounding the delivery layer, the SES-active reporter and the SES-active surface; and
wherein the delivery layer comprises at least one of a thermally depolymerizable polymer, a photoacid generator, a photosensitive compound and a photosensitive polymer.

2. The programmable SES particle of claim 1 further comprising a nanoparticulate SES-active surface.

3. A programmable SES particle comprising
a surface-enhanced spectroscopy (SES)-active surface; and
a programmable reporter associated with the SES surface;
wherein the programmable reporter comprises:
a SES-active reporter associated with the SES-active surface;
a delivery layer;
a diffusion layer associated with the delivery layer and at least one of the SES-active reporter and the SES-active surface; and
an outer shell surrounding the delivery layer, the diffusion layer and the SES-active reporter and the SES-active surface;
wherein the delivery layer comprises at least one of a thermally depolymerizable polymer, a photoacid generator, a photosensitive compound and a photosensitive polymer.

4. A method of marking a material comprising:
providing a material for marking; and
associating a programmable SES particle according to any one of claims 1 to 3 with the material.

5. The method of marking a material of claim 4 wherein the material comprises at least one of a document, a tangible object, a substance, a solid, a fluid and a product.

6. The method of marking a material of claim 4 or claim 5, further comprising obtaining at least one spectroscopic signal by the spectroscopic interrogation of the programmable SES particle; wherein the spectroscopic signal from the programmable SES particle changes over time.

## Patentansprüche

1. Eine programmierbare SES-Partikel, umfassend:
eine bei der oberflächenverstärkten Spektroskopie (SES) aktive Oberfläche; und
einen mit der SES-Oberfläche verbundenen, programmierbaren Reporter;
wobei der programmierbare Reporter umfasst:
einen mit der SES-aktiven Oberfläche verbundenen, SES-aktiven Reporter;
eine Zustellungsschicht, die mit dem SES-aktiven Reporter und der SES-aktiven Oberfläche verbunden ist; und
eine äußere Hülle, die die Zustellungsschicht, den SES-aktiven Reporter und die SES-aktive Oberfläche umgibt; und
wobei die Zustellungsschicht mindestens ein thermisch depolymerisierbares Polymer, einen Photosäure-Erzeuger, eine photoempfindliche Verbindung und ein photoempfindliches Polymer umfasst.

2. Die programmierbare SES-Partikel nach Anspruch 1, weiter umfassend eine nanopartikuläre SES-aktive Oberfläche.

3. Eine programmierbare SES-Partikel, umfassend
eine bei der oberflächenverstärkten Spektroskopie (SES) aktive Oberfläche; und
einen mit der SES-Oberfläche verbundenen, programmierbaren Reporter;
wobei der programmierbare Reporter umfasst:
einen mit der SES-aktiven Oberfläche verbundenen, SES-aktiven Reporter;
eine Zustellungsschicht;
eine Diffusionsschicht, die mit der Zustellungsschicht und mit mindestens dem SES-aktiven Reporter und der SES-aktiven Oberfläche verbunden ist; und
eine äußere Hülle, die die Zustellungsschicht, die Diffusionsschicht und den SES-aktiven Reporter und die SES-aktive Oberfläche umgibt;
wobei die Zustellungsschicht mindestens ein thermisch depolymerisierbares Polymer, einen Photosäure-Erzeuger, eine photoempfindliche Verbindung und ein photoempfindliches Polymer umfasst.

4. Ein Verfahren zum Markieren eines Materials, umfassend:
Bereitstellung eines Materials zum Markieren; und
Verbinden einer programmierbaren SES-Partikel nach einem der Ansprüche 1 bis 3 mit dem Material.

5. Das Verfahren zum Markieren eines Materials nach Anspruch 4, wobei das Material ein Dokument, ein materielles Objekt, eine Substanz, einen Feststoff, ein Fluid und/oder ein Produkt umfasst.

6. Das Verfahren zum Markieren eines Materials nach Anspruch 4 oder Anspruch 5, weiter umfassend das Erhalten mindestens eines spektroskopischen Signals durch die spektroskopische Abfrage der programmierbaren SES-Partikel; wobei sich das spektroskopische Signal von der programmierbaren SES-Partikel mit der Zeit ändert.

## Revendications

1. Une particule programmable SES comprenant:
une surface active de spectroscopie exaltée de surface (SES); et
un dispositif de signalisation programmable associé à la surface SES;
dans laquelle le dispositif de signalisation programmable comprend:
dispositif de signalisation active SES associé à la surface active SES,
une couche de distribution associée à au moins un des éléments suivants: le dispositif de signalisation active SES et la surface active SES; et
une enveloppe extérieure entourant la couche de distribution, le dispositif de signalisation active SES et la surface active SES; et
dans laquelle la couche de distribution comprend au moins un des éléments suivants: un polymère thermiquement dépolymérisable, un générateur de photoacide, un composé photosensible et un polymère photosensible.

2. La particule programmable SES selon la revendication 1, comprenant en outre une surface nanoparticulaire active SES.

3. Une particule programmable SES comprenant:
une surface active de spectroscopie exaltée de surface (SES); et
un dispositif de signalisation programmable associé à la surface SES;
dans laquelle le dispositif de signalisation programmable comprend:
un dispositif de signalisation active SES associé à la surface active SES;
une couche de distribution;
une couche de diffusion associée à la couche de distribution et au moins un des éléments suivants: le dispositif de signalisation active SES et la surface active SES; et
une enveloppe extérieure entourant la couche de distribution, la couche de diffusion et le dispositif de signalisation active SES et la surface active SES;
dans laquelle la couche de distribution comprend au moins un des éléments suivants: un polymère thermiquement dépolymérisable, un générateur de photoacide, un composé photosensible et un polymère photosensible.

4. Un procédé de marquage d'un matériau, comprenant les étapes consistant à:
fourniture d'un matériau pour marquage; et
associer une particule programmable SES selon l'une quelconque des revendications 1 à 3 au matériau.

5. Le procédé de marquage d'un matériau selon la revendication 4, dans lequel le matériau comprend au moins un des éléments suivants: un document, un objet tangible, une substance, un solide, un fluide et un produit.

6. Le procédé de marquage d'un matériau selon la revendication 4 ou 5, comprenant en outre l'étape consistant à obtenir au moins un signal spectroscopique par une interrogation spectroscopique de la particule programmable SES ; dans lequel le signal spectroscopique à partir de la particule programmable SES change avec le temps.
